# FASCICULE DE BREVET EUROPEEN

(11) **EP 0 471 821 B1**
(45) Date de publication et mention de la délivrance du brevet: **08.06.1994**
(21) Numéro de dépôt: 91905833.9
(22) Date de dépôt: 04.03.1991
(51) Int. Cl.: A61F 2/44, A61F 2/46

(54) **PROTHESE POUR DISQUES INTERVERTEBRAUX ET SES INSTRUMENTS D'IMPLANTATION**
BANDSCHEIBENPROTHESE UND INSTRUMENTE FÜR IHRE IMPLANTATION
PROSTHESIS FOR INTERVERTEBRAL DISCS AND INSTRUMENTS FOR ITS IMPLANTATION

(30) Priorité: 07.03.1990 FR 9002997
(43) Date de publication de la demande: 26.02.1992
(73) Titulaire: J.B.S. S.A., F-10000 Troyes (FR)
(72) Inventeur: MARNAY, Thierry, F-30870 Clarensac (FR)
(74) Mandataire: Gérardin, Robert Jean René
(86) Numéro de dépôt international: FR9100173
(87) Numéro de publication internationale: WO9113598

(56) Documents cités:
- EP-A- 0 333 990
- DE-A-22 638 42
- DE-A-30 233 53

## Description

L'invention concerne une prothèse pour disques intervertébraux, destinée à être substituée aux disques fibrocartilagineux assurant la liaison entre les vertèbres de la colonne vertébrale, et ses instruments d'implantation.

Il est bien connu que les disques intervertébraux finissent souvent par se tasser, se déformer, se déplacer, voire s'user tout simplement. Il en résulte des troubles pathologiques multiples, provoquant des douleurs intenses et une gêne certaine pour les patients.

Pendant longtemps, les seules possibilités d'intervention des chirurgiens, destinées principalement à soulager la douleur des patients, consistaient en l'ablation simple du disque déficient ou en un blocage intervertébral ; ce qui soulageait temporairement le patient en lui occasionnant, parfois, un handicap fonctionnel certain. Aussi, au cours des vingt dernières années, a-t'on recouru, avec plus ou moins de succès, à des prothèses destinées à être substituées aux disques intervertébraux dont on effectuait l'ablation totale ou partielle.

Deux voies de recherche ont été suivies dans ce domaine : l'une consistant à remplacer le disque défectueux par un disque à rotule en matière déformable, mais incompressible, disposé entre deux plateaux à empreinte sphérique correspondante, en matière indéformable et incompressible, comme cela est décrit dans le brevet d'invention français 2.372.622 ; l'autre consistant à substituer, au disque défectueux, un disque artificiel en matériau composite offrant sensiblement les mêmes caractéristiques mécaniques que le disque naturel, comme cela est décrit dans le brevet d'invention français n° 2.124.815, à savoir un disque en matière élastomère renforcée par une matière textile.

La combinaison de ces deux voies de recherche a donné naissance à une prothèse, telle que décrite dans la demande de brevet européen n° 0.042.271, ayant principalement pour objet une prothèse bi-composants comportant, respectivement, un bossage hémisphérique et une empreinte hémisphérique ; l'un des composants étant métallique et l'autre synthétique (polyéthylène, polymétacrylate).

Un autre exemple de prothèses de l'art antérieur est décrit dans EP-A-0 333 990.

Si ces prothèses de disques permettent facilement de réintroduire, et de conserver, dans le temps, un écartement normal entre les vertèbres (7 à 14 mm.), leur maintien en position transversale laisse à désirer, car, au cours du déplacement relatif des vertèbres concernées, il se produit un désalignement progressif de la prothèse, entraînant des troubles pathologiques exigeant une intervention chirurgicale rapide. C'est principalement pour tenter de résoudre ce problème que certaines prothèses de disques ont été dotées, sur la face extérieure de leurs plateaux de pression, de petits crampons, destinés à venir s'implanter dans chacune des vertèbres dès que le chirurgien relâche la force d'écartement initialement appliquée, en vue d'obtenir un écartement suffisant des vertèbres pour assurer la mise en place de la dite prothèse. Toutefois, ce mode de solidarisation latérale n'est efficace que lorsque les vertèbres restent parallèles et, par conséquent, lorsque la colonne vertébrale ne subit aucun fléchissement. On comprend qu il ne peut en être de même dans le cas contraire, car, dans ce cas, les crampons restent bien en appui dans leurs empreintes du côté du fléchissement, alors qu'ils s'en échappent du côté opposé ; ce qui provoque un décalage progressif des empreintes lorsque le fléchissement s'accompagne d'une certaine rotation. En conséquence, la prothèse s'éloigne progressivement de son emplacement idéal pour être finalement expulsée en provoquant des troubles graves.

Par ailleurs, il est difficile de concilier, au niveau de la rotule, les caractéristiques de souplesse et d'incompressibilité, lesquelles s accompagnent souvent d'une certaine sensibilité à l'usure et au fluage ; ce qui a obligé, jusqu'à présent, certains fabricants de prothèses pour disques intervertébraux à recourir à des ensembles en trois parties, permettant l'introduction d'une double rotule constituée de deux calottes sphériques à grand rayon, opposées par la base. Cependant, cette conception, bien qu'elle permette d'accroître notablement les surfaces de contact, présente l'inconvénient de réduire considérablement l'épaisseur de la double rotule et de faciliter son désemboîtement, par éviction, en particulier lorsque la colonne vertébrale fléchit au niveau des vertèbres concernées, car cette sollicitation se traduit par un pincement de la double rotule dans le sens du fléchissement, alors que l'écartement est accentué dans l'autre sens.

La présente invention a pour but de remédier à ces inconvénients. Cette invention, telle que décrite dans la revendication 1 résout le problème consistant à créer une prothèse de disques intervertébraux avec laquelle, d'une part, la poussée de la vertèbre supérieure sur la vertèbre inférieure soit transmise, quelle que soit l'inclinaison relative des deux vertèbres, par l'intermédiaire d'éléments à large surface d'appui, offrant une bonne résistance à l'usure et conférant, à l'ensemble, une certaine souplesse tout en contrôlant le débattement angulaire longitudinal et transversal relatif des vertèbres concernées. Les éléments supérieur et inférieur de la prothèse étant, par ailleurs, suffisamment ancrés dans leur plateau de vertèbres respectif pour éviter tout risque de désassemblage ; ceci sans que les vertèbres en soient fragilisées et en faisant appel à une instrumentation simple, facile à mettre en oeuvre.

La prothèse pour.disques intervertébraux selon l'invention se caractérise, principalement, en ce qu'elle est constituée de deux plateaux, munis chacun d'ailerons d'ancrage, séparés par un organe d'articulation constitué d'une calotte sphérique à base cylindrique, implantée à force dans une cavité cylindrique de même diamètre réalisée dans la face supérieure du plateau inférieur, et en ce que chacun des plateaux comporte, sur l'arrière, des orifices filetés disposés symétriquement de part et d'autre de la cavité cylindrique et parallèlement aux ailerons d'ancrage.

Le débattement angulaire des plateaux inférieur et supérieur, l'un par rapport à l'autre, est limité à une valeur alpha par deux collerettes à double redans, de sections complémentaires, situées respectivement sur le pourtour du plateau supérieur et sur le pourtour du plateau inférieur.

La collerette à double redans, réalisée dans le plateau inférieur, occupe toute la partie située entre le bord de la cavité d'implantation de l'organe d'articulation et le bord du dit plateau, alors que la collerette à double redans, réalisée dans le plateau supérieur, occupe toute la partie située entre le bord de la cavité semi-sphérique assurant la portée de l'organe d'articulation et le bord du dit plateau.

Le diamètre (D) de la cavité semi-cylindrique, assurant la portée de l'organe d'articulation, et les caractéristiques géométriques des collerettes à double redans, supérieure et inférieure, sont déterminés pour que, lorsque les dites collerettes viennent s'imbriquer l'une dans l'autre, le décalage angulaire relatif alpha maximum des plateaux corresponde à celui qui existe naturellement entre deux vertèbres, à savoir 15° environ.

Le double redans du plateau supérieur est constitué de la jonction d'une surface annulaire, inclinée vers la base du dit plateau selon un angle bêta d'enuiron 10° à partir du bord de celui-ci, et d'une autre surface annulaire partant du bord de la cavité semi-sphérique et formant un angle delta d'environ 70° par rapport à la base du plateau.

La collerette à double redans du plateau inférieur est constituée de la jonction d'une surface annulaire, relevée d'un angle téta d'environ 20° par rapport à la base du dit plateau, et d'une autre surface annulaire partant du bord de la cavité cylindrique et formant un angle gamma d'environ 60° par rapport à la base du plateau.

Selon un mode de réalisation préférentiel, la base de l'organe d'articulation prend appui, dans le fond de sa cavité cylindrique d'implantation, par l'intermédiaire d'une rondelle, en matériau souple, de diamètre (d) inférieur à celui (D) de l'organe d'articulation ; le dit organe d'articulation est en polyéthylène, les plateaux sont réalisés en titane, ou alliage de titane, et leurs surfaces de contact avec les vertèbres reçoivent préalablement un traitement au plasma, destiné à faciliter l'ostéosynthèse.

Les ailerons d'ancrage des plateaux dans la base des vertèbres sont effilés et comportent, à leur extrémité, un relief en "dents de scie" dont l'arête supérieure des dents est inclinée vers l'avant dans le sens de l'introduction des ailerons dans leurs mortaises.

La largeur (ℓ) des mortaises est un peu inférieure à l'épaisseur (E) des ailerons, mesurée à leur base.

Les intruments d'implantation de la prothèse selon l'invention se caractérisent en ce qu'ils sont constitués principalement :
- d'un jeu de gabarits d écartement et de mortaisage de vertèbres,
- d'un ciseau ostéotome pour la réalisation des mortaises,
- d'un embout impacteur de prothèses, avec plaque de frappe incorporée,
- d'un outil de manoeuvre des gabarits, des impacteurs de prothèses et du ciseau ostéotome,
- d'une pince de pose de la prothèse,
- d'un extracteur de prothèses.

comme décrit dans la revendication 14

Les gabarits d'écartement et de mortaisage sont constitués d'une plaque d'épaisseur appropriée, de même forme et de même encombrement que les plateaux correspondants de la prothèse, comportant, vers l'avant, des angles arrondis et, de l'arrière vers l'avant, un trou borgne fileté, de même dimension que l'extrémité filetée de la tige porte-gabarit et impacteur, situé dans le plan médian longitudinal de la plaque, et quatre rainures, opposées deux à deux, disposées symétriquement, de part et d'autre du plan médian longitudinal de la plaque à une distance correspondant exactement à celle séparant les ailerons d'ancrage de chacun des plateaux. La longueur des rainures est déterminée de façon à limiter la pénétration du ciseau ostéotome à une profondeur juste suffisante à l'ancrage et au positionnement exact de chacun des plateaux. Le dit ciseau comportant, par ailleurs, un talon limitant sa pénétration longitudinale, à une profondeur correspondant à la longueur des rainures.
L'embout impacteur de prothèses est constitué d'un élément fileté, de forme parallélépipédique, réalisé dans un matériau tendre, dont l'une des extrémités, opposée au filetage, est conformée selon la forme de la prothèse et dont l'autre extrémité est munie d'une plaque de frappe réalisée dans un matériau dur.

L'outil de manoeuvre des gabarits, de l'embout impacteur de prothèses et du ciseau ostéotome est constitué, principalement, d'une tige métallique, dont l'une des extrémités est filetée aux dimensions de l'orifice taraudé des gabarits de rainurage, de l'embout impacteur et du talon du ciseau ostéotome, et dont I 'autre extrémité comporte un carré d'entraînement, précédé d'une gorge semi- circulaire, sur lequel s'adapte un manche escamotable par l'intermédiaire d'un poussoir, rappelé vers l'extérieur par un ressort, assurant le blocage et le déblocage par l'intermédiaire d'une clavette cylindrique mobile dans un logement oblong. Le blocage et le déblocage étant obtenus par l'intermédiaire de deux rainures tangentielles, de profondeurs différentes, réalisées dans le poussoir.

La pince de pose de prothèses est constituée de branches, normalement maintenues écartées par un ressort, munies chacune d'un embout épousant la forme des plateaux de la prothèse, comportant deux tiges destinées à pénétrer à l'intérieur des orifices prévus à cet effet dans les plateaux.

L'extracteur de prothèses est constitué de quatre tiges parallèles, à bouton de manoeuvre moletté et à extrémité filetée, montées coulissantes dans une plaque de manoeuvre collective, servant aussi de gabarit de montage de l'extrémité filetée des tiges dans les orifices filetés des plateaux de la prothèse, et d'organe de frappe pour le démontage.
Les avantages obtenus, grâce à cette invention, consistent essentiellement en ceci que la prothèse est maintenue en permanence à l'emplacement idéal, sans risque de déplacement, de fluage ni de déboîtement intempestif grâce, notamment, à l'ancrage de chacun des plateaux par l'intermédiaire de deux ailerons qui assurent l'immobilisation en rotation et transversale, en augmentant la surface de contact avec l'os et en évitant toute expulsion postérieure, puisque les mortaises ne franchissent pas la corticale postérieure de la vertèbre, et toute expulsion antérieure, puisque les ailerons comportent, à leurs extrémités, des dents de forme appropriée qui viennent s'implanter dans le fond des rainures, grâce aussi au fait qu'une certaine ostéosynthèse s'effectue avec le métal des plateaux (titane), accentuée par le traitement préalable au plasma, à la limitation du mouvement à environ 15° par les collerettes à redans formant butées et au mode d'implantation de l'organe d'articulation dans le plateau inférieur.

L' implantation de cette prothèse s'effectue à l'aide de moyens simples ne permettant aucune fausse interprétation ni malfaçon dans la réalisation des mortaises d'ancrage et dans la mise en place des plateaux.

D'autres caractéristiques et avantages apparaîtront dans la description qui va suivre d'une prothèse de disques intervertébraux et de ses instruments de mise en place, réalisés selon l'invention, donnés à titre d'exemple non limitatif au regard des dessins annexés sur lesquels :
- la figure 1 représente une vue de face, en coupe longitudinale, de la prothèse, en place entre deux vertèbres représentées en coupe partielle,
- la figure 2 représente une vue de dessus de la prothèse,
- la figure 3 représente une vue de côté de la prothèse, en place entre deux vertèbres représentées en coupe partielle,
- la figure 4 représente une vue de face d'un gabarit de montage et de mortaisage,
- la figure 5 représente une vue de dessus d'un gabarit de montage et de mortaisage, en place à l'extrémité de la tige de l'outil de manoeuvre (en trait mixte),
- la figure 6 représente une vue de côté d'un gabarit de montage et de mortaisage, en place à l'extrémité de la tige de l'outil de manoeuvre (en trait mixte),
- la figure 7 représente une vue de face d'un embout impacteur de prothèses, en place à l'extrémité de la tige de l'outil de manoeuvre (en trait mixte),
- la figure 8 représente une vue en coupe longitudinale de l'outil de manoeuvre,
- la figure 9 représente une vue de dessus, en coupe selon le plan AA, de l'outil de manoeuvre,
- la figure 10 représente une vue de côté de la pince de pose de la prothèse,
- la figure 11 représente une vue de dessus de la pince de pose de la prothèse,
- la figure 12 représente une vue de dessus de l'extracteur de prothèses,
- la figure 13 représente une vue de face de l'extracteur de prothèses,
- la figure 14 représente une vue de dessus du ciseau ostéotome,
- la figure 15 représente une vue de côté du ciseau ostéotome,
- la figure 16 représente une vue de face du ciseau ostéotome.

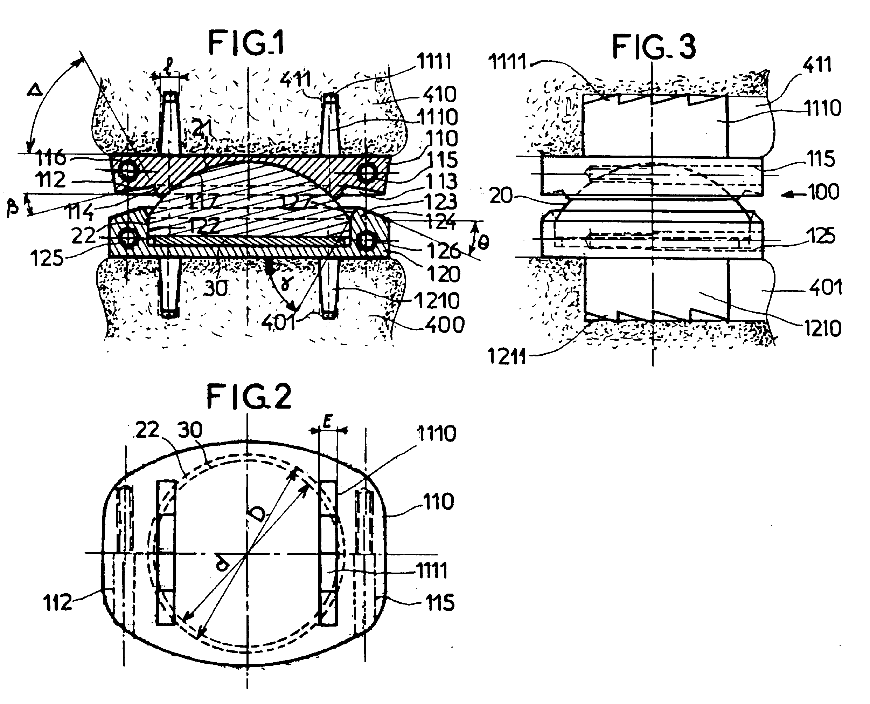

Les figures 1 à 3 représentent une prothèse **100** pour disques intervertébraux **400** et **410** comportant, essentiellement, deux plateaux **110** et **120**, munis chacun d'ailerons d'ancrage **1110**, **1210**, séparés par un organe d'articulation **20** constitué d'une calotte sphérique **21** à base cylindrique **22**, implantée dans une cavité cylindrique **122** de même diamètre (D), réalisée dans la face supérieure du plateau inférieur **120**, avec interposition d'une rondelle souple **30** de diamètre (d) ; les dits plateaux **110**, **120** comportant des orifices filetés **115**, **116** et **125**, **126**, disposés symétriquement de part et d'autre de la cavité cylindrique **122**, et des collerettes à double redans **113** et **123**, de sections complémentaires, constituées, respectivement, de la jonction d'une surface annulaire rentrée **112,** inclinée selon un angle bêta d'environ 10° à partir du bord, et d'une autre surface annulaire **114**, partant du bord, la cavité semi-sphérique **117**, inclinée selon un angle delta d'environ 70° par rapport à la base du plateau supérieur **110**, et de la jonction d'une surface annulaire **124** relevée d'un angle téta d'environ 20° par rapport à la base du plateau inférieur **120**, et d'une autre surface annulaire **127,** partant du bord de la cavité cylindrique **122** et formant un angle gamma d'environ 60° par rapport à la base du plateau inférieur **120**.
Les ailerons d'ancrage **1110** et **1210** sont effilés et comportent, à leur extrémité, un relief en "dents de scie" **1111** et **1211** ; les dents étant inclinées vers l'avant, dans le sens de l'introduction, dans les mortaises **401** et **411** préalablement réalisées, à l'aide d'un ciseau ostéotome **90**, dans les vertèbres **400**, **410** à l'aide du gabarit **50** qui va être maintenant décrit.

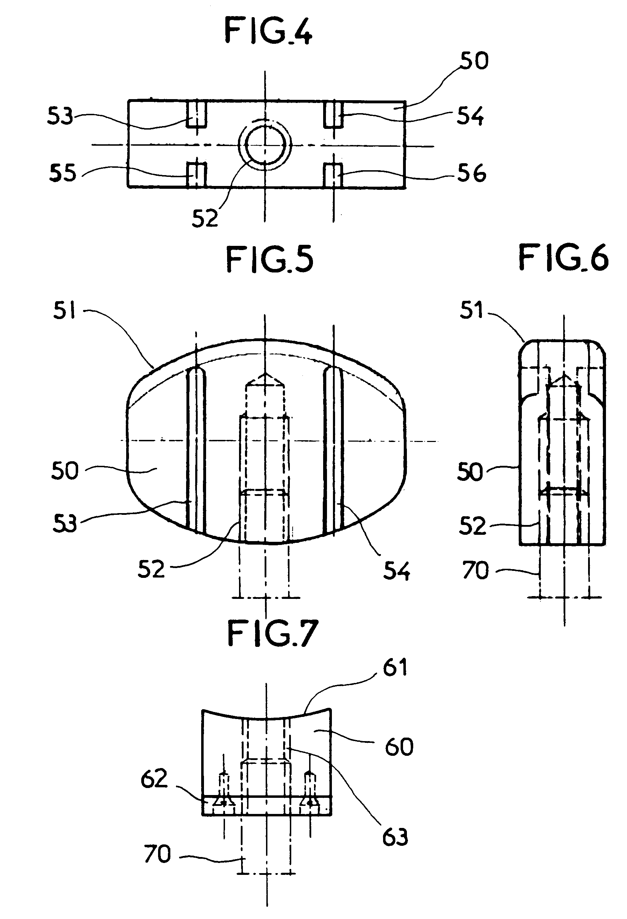

Les gabarits d'écartement et de mortaisage **50** selon l'invention, représentés aux figures 4 et 6, formant un jeu de différentes dimensions, sont constitués d'une plaque de même épaisseur, de même forme et de même encombrement que les plateaux **110** et **120** de la prothèse **100**, comportent, vers l'avant, des angles arrondis **51** et, de l'arrière vers l'avant, un trou borgne fileté **52**, destiné à permettre la solidarisation à l'extrémité filetée **71** de la tige **70** de l'outil de mise en place. Les dits gabarits comportent quatre rainures **53**, **54**, **55**, **56** séparées par une distance correspondant à celle séparant les ailerons d'ancrage **1110** et **1210** des plateaux **110** et **120** ; la longueur des rainures **53**, **54**, **55** et **56** est déterminée de façon à limiter la pénétration du ciseau ostéotome **90** à une profondeur juste suffisante à l'ancrage exact de chacun des plateaux **110**, **120** ; la lame **91** du dit ciseau **90** comportant un talon **92** limitant sa pénétration à la longueur des rainures **53**, **54**, **55**, **56**, dont l'extrémité doit correspondre sensiblement à la corticale postérieure des vertèbres.

L'embout impacteur **60** de prothèses selon l'invention, représenté à la figure 7, est constitué, principalement, d'un élément de forme parallélépipédique, réalisé dans un matériau tendre, dont l'une des extrémités **61** est conformée selon la forme du bord extérieur des plateaux **110**, **120** et dont l'autre extrémité est munie d'un trou fileté **62** permettant le vissage de l'extrémité **71** de la tige **70** de l'outil, et comporte une plaque de frappe rapportée **63**, en matériau dur.

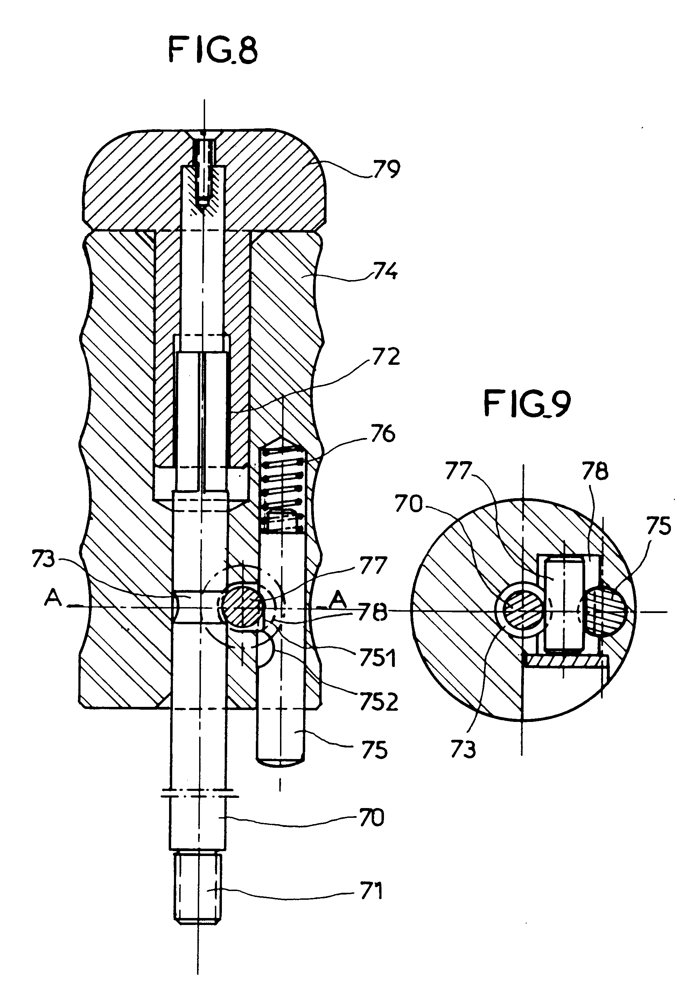

L'outil de manoeuvre des gabarits **50**, de l'embout impacteur **60** de prothèses et du ciseau ostéotome **90**, représenté aux figures 8 et 9, est constitué, principalement, d'une tige métallique **70** dont l'extrémité **71** est filetée aux dimensions des orifices taraudés **52** et **62**, des gabarits **50** et de l'embout impacteur **60**.

L'autre extrémité de cette tige comporte un carré d'entraînement **72** par le manche **74**, lequel s'adapte, à volonté, sur la tige, par l'intermédiaire de la gorge semi-circulaire **73** réalisée sur la tige, sous le carré d'entraînement **72**, et d'une clavette cylindrique **77** mobile dans un logement oblong **78** aménagé dans le manche **74** ; ladite clavette **77** pouvant être, par l'intermédiaire d'un poussoir **75** rappelé vers l'extérieur par un ressort **76**, bloquée dans le fond de la gorge **73** de la tige, ou entièrement libérée de celle-ci selon que l'on aura, ou non, appliqué une pression dans l'axe du dit poussoir **75** ; des empreintes de profondeurs différentes **751** et **752** ayant été aménagées, pour ce faire, tangentiellement au poussoir **75**. Le manche **74** comporte, à sa partie supérieure, une pièce de frappe métallique **79** permettant d'appliquer directement des chocs sur la tige **70**, dans l'axe de celle-ci.

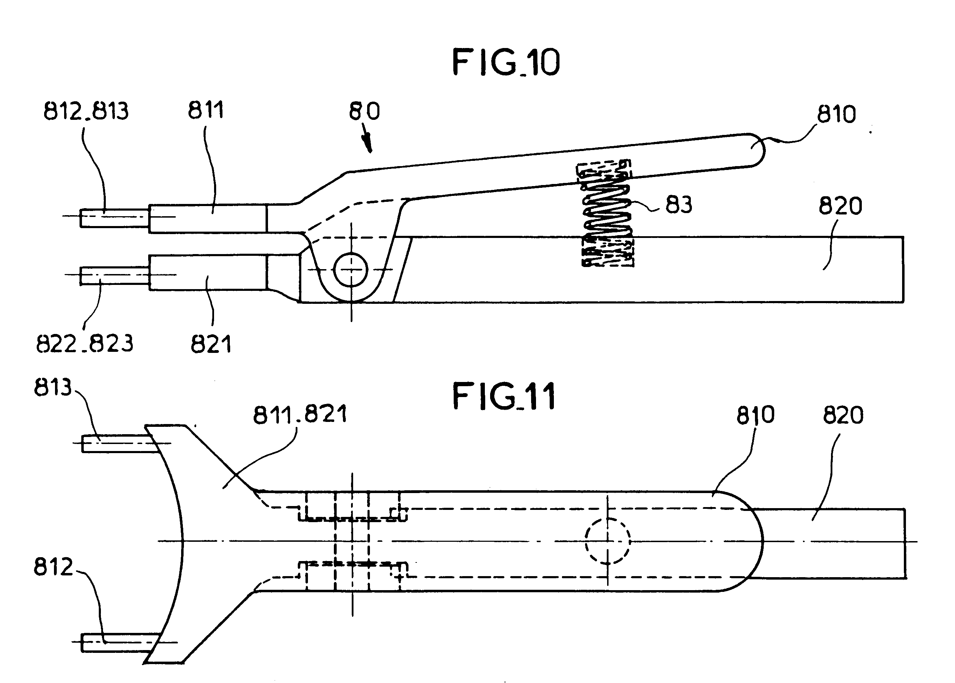

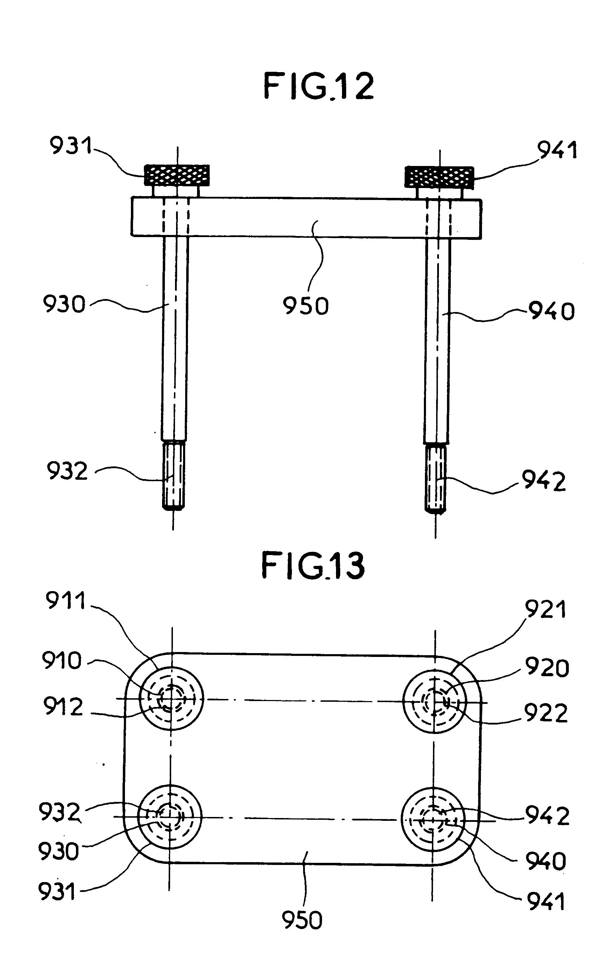

La pince de pose **80** de prothèses, représentée aux figures 10 et 11, est, quant à elle, constituée de deux branches **810** et **820**, maintenues normalement écartées par un ressort **83**, dont les becs sont munis chacun d'un embout **811**, **821** épousant la forme des plateaux **110**, **120** de laprothèse et comportant deux tiges **812**, **813** et **822**, **823** destinées à pénétrer à l'intérieur des orifices **115**, **116** et **125**, **126** prévus à cet effet dans les plateaux. Les dits orifices n'ayant été, en conséquence, filetés que sur une partie seulement de leur profondeur, à partir du fond ; cette longueur filetée ne servant, elle, qu'au retrait, à l'aide de l'extracteur qui va être maintenant décrit.

Cet extracteur **900** de prothèses, représenté aux figures 12 et 13, est constitué de quatre tiges **910**, **920**, **930**, **940**, dont l'une des extrémités **912**, **922**, **932** et **942** est filetée aux dimensions des orifices filetés de la prothèse et dont l'autre extrémité comporte des boutons moletés **911**, **921**, **931**, **941**, montés coulissants dans une plaque **950** formant traverse et organe de frappe.

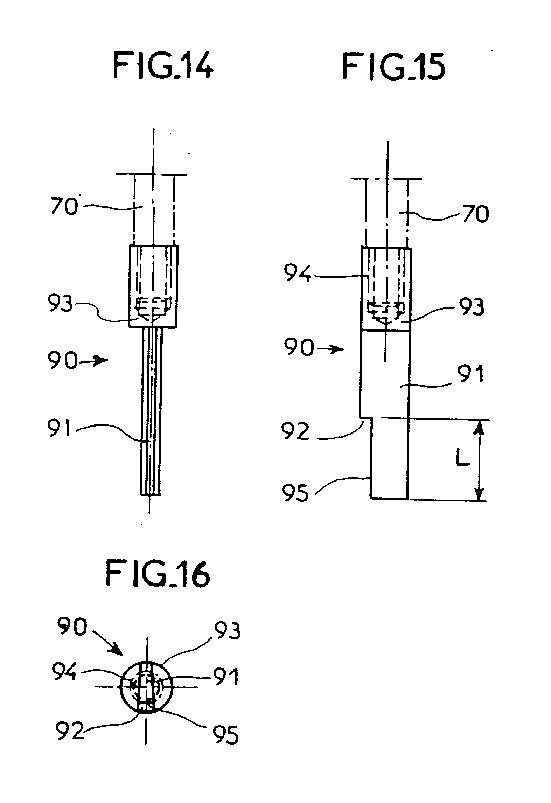

En se rapportant maintenant aux figures 14, 15 et 16, on remarque que le ciseau ostéotome **90** est constitué, principalement, d'une embase **93** à orifice taraudé **94** aux dimensions de l'extrémité filetée **71** de la tige **70** de l'outil de manoeuvre, prolongée par une lame **91** à talon limiteur de pénétration **92**. La lame **91** est effilée vers le bord opposé au talon **92**. La longueur (L) du dos **95** de la partie active de la lame est un peu inférieure à l'épaisseur (E) des ailerons **1110**, **1210**, mesurée à leur base ; ceci afin d'obtenir un certain pincement après mise en place dans les mortaises **401**, **411** réalisées en utilisant le gabarit **50** et le ciseau ostéotome **90**.

Le procédé de mise en oeuvre des instruments d'implantation de la prothèse pour disques intervertébraux selon l'invention consiste à effectuer, dans l'ordre, les opérations suivantes :
- élimination du disque intervertébral défectueux, en utilisant les méthodes et les instruments classiques,
- écartement des vertèbres pour permettre l'insertion d'un gabarit **50**, choisi en fonction des particularités propres des vertèbres concernées **400**, **410**,
- réalisation des mortaises **401**, **411** destinées à permettre l'ancrage des plateaux **110, 120** dans les vertèbres **400, 410**, par l'intermédiaire des ailerons **1110, 1210**. Ce mortaisage s'effectue en utilisant le ciseau ostéotome **90**, qui aura été, pour ce faire, vissé sur l'extrémité filetée **71** de la tige de l'outil de manoeuvre ; le dit ciseau étant introduit successivement dans les rainures **53, 54, 55** et **56**, en présentant la lame **91** de celui-ci, dos **95** contre le fond des dites rainures, afin que la partie effilée soit dirigée vers la vertèbre **400** ou **410** concernée et que le talon **92** de la lame s'oppose à la pénétration de celle-ci au-delà de la longueur strictement nécessaire à l'implantation des ailerons d'ancrage **1110**, **1210**. Cette longueur ayant été déterminée pour que les ailerons viennent buter contre la corticale postérieure de la vertèbre, afin d'éliminer tout risque d'expulsion postérieure de la prothèse,
- retrait du gabarit **50**, après réalisation de toutes les mortaises supérieures et inférieures, tout en maintenant l'écartement des vertèbres **400**, **410** pour permettre la mise en place de la prothèse **100**,
- mise en place de la prothèse **100** entre les vertèbres **400**, **410**, en utilisant la pince de pose **80** dont les quatre tiges **812**, **813** et **822, 823** ont été préalablement introduites dans les orifices **115**, **116** et **125**, **126** des plateaux **110** et **120** de prothèses. L'introduction complète des ailerons **1110, 1210** dans leursmortaises pouvant être facilitée en exerçant une pression, ou des chocs, à l'extrémité de l'une des branches **820** de la pince, qui a été prolongée et conformée, pour ce faire, en disposant, notamment, l'embout **821**, portant les tiges **822**, **823**, dans le prolongement de la dite branche **820,**
- relâchement de l'effort d'écartement, exercé sur les vertèbres **400**, **410**, pour obtenir la mise en appui totale de l'organe d'articulation **20** dans la cavité semi-sphérique **117** du plateau supérieur **110**, des plateaux supérieur et inférieur **110** et **120** contre les vertèbres **400**, **410** et de l'extrémité des dents **1111** des ailerons **1110** et **1210** contre le fond des mortaises **401** et **411** ; ce qui, compte-tenu de la forme des dents, élimine tout risque d'expulsion antérieure de la prothèse.

Certaines circonstances peuvent amener à extraire la prothèse en vue de son remplacement ; lequel pourra être effectué en procédant comme suit :
- écartement des vertèbres **400**, **410** à l'aide d'un écarteur classique, afin de supprimer la pression exercée sur la prothèse et de faciliter le décollement de ses plateaux **110, 120,**
- montage de l'extracteur **900** de prothèses par vissage des extrémités filetées des tiges **910, 920, 930** et **940**, après mise en place de celles-ci dans les orifices prévus à cet effet dans la plaque de liaison **950**, dans les orifices taraudés **115**, **116**, **125**, **126** existant dans les plateaux **110** et **120** de la prothèse **100**, afin de pouvoir, en exerçant des chocs sous les boutons moletés **911, 921, 931** et **941**, par intermédiaire de la plaque **950**, obtenir le décollement des plateaux et le désengagement des ailerons **1110**, **1210** de leurs mortaises **401**, **411**.
- la remise en place de la prothèse de remplacement s'effectuera en procédant comme indiqué ci-dessus pour la mise en place de la prothèse d'origine.

La prothèse pour disques intervertébraux et ses instruments d'implantation selon l'invention est destinée, principalement, à la chirurgie vertébrale.

## Revendications

1. Prothèse pour disques intervertébraux, caractérisée en ce qu'elle est constituée principalement de deux plateaux (**110**, **120**), munis chacun d'ailerons d'ancrage (**1110**, **1210**), séparés par un organe d'articulation (**20**) constitué d'une calotte sphérique (**21**) caractérisée en ce que la calotte sphérique (**21**) a une base cylindrique (**22**) implantée dans une cavité cylindrique (**122**) de même diamètre, réalisée dans la face supérieure du plateau inférieur (**120**) et en ce que chacun des plateaux (**110**, **120**) comporte, sur l'arrière, des orifices filetés (**115**, **116**, **125, 126**), disposés symétriquement de part et d'autre de la cavité cylindrique (**122**) et parallèlement aux ailerons d'ancrage (**1110**, **1210**).

2. Prothèse selon la revendication 1, caractérisée en ce que le débattement angulaire des plateaux inférieur (**120**) et supérieur (**110**), l'un par rapport à l'autre, est limité par deux collerettes à double redans (**113**, **123**), de sections complémentaires, situées respectivement sur le pourtour du plateau supérieur (**110**) et sur le pourtour du plateau inférieur (**120**).

3. Prothèse selon la revendication 2, caractérisée en ce que la collerette à double redans (**123**), réalisée dans le plateau inférieur (**120**), occupe toute la partie située entre le bord de la cavité cylindrique (**122**) de l'organe d'articulation (**20**) et le bord du dit plateau (**120**).

4. Prothèse selon la revendication 2, caractérisée en ce que la collerette à double redans (**113**), réalisée dans le plateau supérieur (**110**), occupe toute la partie située entre le bord de la cavité semi-sphérique (**117**), assurant la portée de l'organe d'articulation (**20**), et le bord du dit plateau (**110**).

5. Prothèse selon la revendication 4, caractérisée en ce que le diamètre (D) de la cavité cylindrique (**122**), assurant la portée de l'organe d'articulation (**20**), et les caractéristiques géométriques des collerettes à double redans supérieure (**113**) et inférieure (**123**) sont déterminés pour que, lorsque les dites collerettes (**113, 123**) viennent s'imbriquer l'une dans l'autre, le décalage angulaire relatif alpha maximum des plateaux (**110**, **120**) corresponde à celui qui existe naturellement entre deux vertèbres.

6. Prothèse selon la revendication 3, caractérisée en ce que là collerette à double redans (**113**) du plateau supérieur (**110**) est constituée de la jonction d'une surface annulaire (**112**), inclinée vers la base du dit plateau (**110**) selon un angle bêta d'environ 10° à partir du bord de celui-ci, et d'une autre surface annulaire (**114**), partant du bord de la cavité semi-sphérique (**117**), et formant un angle delta d'environ 70° par rapport à la base du plateau (**110**).

7. Prothèse selon la revendication 4, caractérisée en ce que la collerette à double redans (**123**) du plateau inférieur (**120**) est constituée de la jonction d'une surface annulaire (**124**), relevée d'un angle téta d'environ 20° par rapport à la base du dit plateau (**120**), et d'une autre surface annulaire (**127**), partant du bord de la cavité cylindrique (**122**), et formant un angle gamma d'environ 60° par rapport à la base du plateau (**120**).

8. Prothèse selon la revendication 1, caractérisée en ce que la base (**22**) de l'organe d'articulation (**20**) prend appui, dans le fond de sa cavité cylindrique (**122**), par l'intermédiaire d'une rondelle, en matériau souple (**30**), de diamètre (d) inférieur à celui (D) de la base (**22**) de l'organe d'articulation (**20**).

9. Prothèse selon la revendication 1, caractérisée en ce que les ailerons d'ancrage (**1110, 1210**) des plateaux (**110, 120**) dans la base des vertèbres (**400**, **410**) sont effilés et comportent, à leur extrémité, un relief en "dents de scie" (**1111**, **1211**).

10. Prothèse selon la revendication 9, caractérisée en ce que l'arête supérieure des dents est inclinée vers l'avant, dans le sens de l'introduction des ailerons dans leurs mortaises (**401**, **411**).

11. Prothèse selon la revendication 1 ou 9, caractérisée en ce que l'épaisseur (E) des ailerons (**1110**, **1210**), à leur base, est un peu supérieure à la largeur (ℓ) des mortaises (**401**, **411**) préalablement réalisées dans les vertèbres (**400**, **410**).

12. Prothèse selon la revendication 1, caractérisée en ce que les plateaux (**110**, **120**) et leurs ailerons (**1110**, **1210**) sont réalisés en titane, ou en alliage de titane, traité superficiellement au plasma sur toutes les surfaces venant au contact des vertèbres.

13. Prothèse selon la revendication 1, caractérisée en ce que l'organe d'articulation (**20**) est réalisé en polyéthylène.

14. Instruments d'implantation de la prothèse selon l'une quelconque des revendications ci-dessus comprenant une pince de pose de la prothèse (80), caractérisés en ce qu'ils sont constitués :
- d'un jeu de gabarits (**50**) d'écartement et de mortaisage de vertèbres,
- d'un ciseau ostéotome (**90**) destiné à la réalisation des mortaises (**401**, **411**),
- d'un embout impacteur de prothèses (**60**), avec plaque de frappe incorporée (**63**),
- d'un outil de manoeuvre des gabarits (**50**), des impacteurs de prothèses (**60**) et du ciseau ostéotome (**90**),
- d'un extracteur de prothèses (**900**).

15. Instruments d'implantation selon la revendication 14, caractérisés en ce que les gabarits d'écartement et de mortaisage (**50**) sont constitués d'une plaque, d'épaisseur appropriée, de même forme et de même encombrement que les plateaux (**110**, **120**) correspondants de la prothèse (**100**), comportant, vers l'avant, des angles arrondis (**51**) et, de l'arrière vers l'avant, un trou borgne fileté (**52**), de même dimension que l'extrémité filetée (**71**) de la tige (**70**) de l'outil de manoeuvre des gabarits (**50**) et de l'impacteur (**60**), situé dans le plan médian longitudinal de la plaque, et quatre rainures (**53, 54, 55, 56**), opposées deux à deux, disposées symétriquement de part et d'autre du plan médian longitudinal de la plaque, à une distance correspondant exactement à celle séparant les ailerons d'ancrage (**1110**, **1210**) de chacun des plateaux (**110**, **120**).

16. Instruments selon la revendication 15, caractérisés en ce que la longueur des rainures (**53, 54, 55, 56**) est déterminée de façon à limiter la pénétration du ciseau ostéotome (**90**) à une profondeur juste suffisante à l'ancrage et au positionnement exact de chacun des ailerons (**1110**, **1210**) correspondant à la corticale postérieure de la vertèbre.

17. Instruments selon la revendication 14, caractérisés en ce que l'embout impacteur (**60**) de prothèses est constitué d'un élément fileté, de forme parallélépipédique, en matériau tendre, dont l'une des extrémités, opposée au filetage (**62**), est conformée selon la forme des plateaux (**110**, **120**) de la prothèse, et dont l'autre extrémité est munie d'une plaque de frappe (**63**), en matériau dur.

18. Instruments selon la revendication 14, caractérisés en ce que l'outil de manoeuvre des gabarits, de l'embout impacteur de prothèses et du ciseau ostéotome est constitué principalement d'une tige métallique (**70**), dont l'une des extrémités (**71**) est filetée aux dimensions de l'orifice taraudé des gabarits de mortaisage, de l'embout impacteur et de l'embase (**93**) du ciseau (**90**), et dont l'autre extrémité comporte un carré d'entraînement (**72**), précédé d'une gorge semi-circulaire (**73**), sur lequel s'adapte un manche (**74**), à embout de frappe (**79**), escamotable par l'intermédiaire d'un poussoir (**75**) rappelé par un ressort (**76**) assurant, par l'intermédiaire d'une clavette cylindrique mobile (**77**) emprisonnée dans un logement oblong (**78**), la solidarisation du dit manche (**74**) à la tige (**70**), par l'intermédiaire de la gorge semi-circulaire (**73**) et de rainures tangentielles (**751**, **752**), de profondeurs différentes, réalisées dans le poussoir (**75**).

19. Instruments selon la revendication 14, caractérisés en ce que la pince de pose (**80**) de la prothèse (**100**), dont les branches (**810, 820**) sont normalement écartées par un ressort (**83**), est munie d'embouts (**811, 821**) épousant la forme des plateaux (**110**, **120**) de la prothèse (**100**), comportant chacun deux tiges (**812, 813** et **822, 823**), destinées à pénétrer à l'intérieur des orifices (**115, 116,** **125, 126**) prévus à cet effet dans les plateaux (**110**, **120**).

20. Instruments selon la revendication 14, caractérisés en ce que l'extracteur de prothèses est constitué de quatre tiges (**910**, **920**, **930**, **940**), à bouton de manoeuvre moleté (**911, 921, 931, 941**) et à extrémité filetée (**912**, **922, 932, 942**), montées coulissantes dans des orifices percés dans une plaque de manoeuure collective (**950**) servant de gabarit de positionnement par rapport aux orifices filetés prévus à cet effet dans les plateaux, et d'organe de frappe.

## Claims

1. Prosthesis for intervertebral discs, characterised in that it consists principally of two plates (110, 120), each having anchoring fins (1110, 1210) and separated by an articulation member (20) consisting of a spherical dome (21), characterised in that the spherical dome (21) has a cylindrical base (22) located in a cylindrical cavity (122) of the same diameter and made in the top face of the bottom plate (120), and in that each of the plates (110, 120) has, at its rear, threaded orifices (115, 116, 125, 126) disposed symmetrically on each side of the cylindrical cavity (122) and parallel to the anchoring fins (1110, 1210).

2. Prosthesis according to Claim 1, characterised in that the angular movement of the bottom (120) and top plates (110), with respect to each other, is limited by two double-stepped rings (113, 123) with complementary cross sections, located respectively on the circumference of the top plate (110) and on the circumference of the bottom plate (120).

3. Prosthesis according to Claim 2, characterised in that the double-stepped ring (123) made in the bottom plate (120) occupies the whole of the part located between the edge of the cylindrical cavity (122) of the articulation member (20) and the edge of the said plate (120).

4. Prosthesis according to Claim 2, characterised in that the double-stepped ring (113) made in the top plate (110) occupies the whole of the part located between the edge of the semi-spherical cavity (117) bearing the articulation member (20), and the edge of the said plate (110).

5. Prosthesis according to Claim 4, characterised in that the diameter (D) of the cylindrical cavity (122) bearing the articulation member (20) and the geometric characteristics of the top (113) and bottom (123) double-stepped rings are determined so that, when the said rings (113, 123) fit into each other, the maximum relative angular separation alpha of the plates (110, 120) corresponds to that which exists naturally between two vertebrae.

6. Prosthesis according to Claim 3, characterised in that the double-stepped ring (113) on the top plate (110) is formed by the joining of an annular surface (112) sloping towards the base of the said plate (110) at an angle beta of about 10° from the edge of the latter, and another annular surface (114) starting at the edge of the semi-spherical cavity (117) and forming an angle delta of approximately 70° with respect to the base of the plate (110).

7. Prosthesis according to Claim 4, characterised in that the double-stepped ring (123) on the bottom plate (120) is formed by the joining of an annular surface (124) raised at an angle theta of approximately 20° with respect to the base of the Osaid plate (120), and another annular surface (127) starting from the edge of the cylindrical cavity (122) and forming an angle gamma of approximately 60° with respect to the base of the plate (120).

8. Prosthesis according to Claim 1, characterised in that the base (22) of the articulation member (20) bears on the base of its cylindrical cavity (122) through a washer made of flexible material (30) and the diameter (d) of which is less than the diameter (D) of the base (22) of the articulation member (20).

9. Prosthesis according to Claim 1, characterised in that the fins (1110, 1210) for anchoring the plates (110, 120) in the base of the vertebrae (400, 410) are tapered and have, at their end, a "saw-tooth" shape (1111, 1211).

10. Prosthesis according to Claim 9, characterised in that the top ridge of the teeth slopes forwards, in the direction of the insertion of the fins into their slots (401, 411).

11. Prosthesis according to Claim 1 or 9, characterised in that the thickness (E) of the fins (1110, 1210), at their base, is slightly greater than the width (1) of the slots (401, 411) previously formed in the vertebrae (400, 410).

12. Prosthesis according to Claim 1, characterised in that the plates (110, 120) and their fins (1110, 1210) are made of titanium, or an alloy of titanium, surface-treated with plasma on all the surfaces coming into contact with the vertebrae.

13. Prosthesis according to Claim 1, characterised in that the articulation member (20) is made of polyethylene.

14. Instruments for the implantation of the prosthesis according to any of the above claims, comprising forceps for fitting the prosthesis (80 ), characterised in that they consist of:
- a set of jigs (50) for separating and cutting slots in vertebrae,
- an osteotome chisel (90) intended to cut the slots (401, 411),
- a prosthesis impacter connector (60), with a built-in strike plate (63),
- a tool for manoeuvring the jigs (50), the prosthesis impacters (60) and the osteotome chisel (90),
- a prosthesis extractor (900).

15. Implantation instruments according to Claim 14, characterised in that the separating and slot-cutting jigs (50) consist of a plate, of appropriate thickness, of the same shape and the same dimensions as the corresponding plates (110, 120) of the prosthesis (100), having, towards the front, rounded corners (51) and, from the rear towards the front, a threaded blind hole (52) of the same dimensions as the threaded end (71) of the rod (70) of the tool for manoeuvring the jigs (50) and the impacter (60), situated in the longitudinal mid-plane of the plate, and four grooves (53, 54, 55, 56), opposite each other in pairs, disposed symmetrically on each side of the longitudinal mid-plane of the plate at a distance which corresponds exactly to the distance separating the anchoring fins (1110, 1210) of each of the plates (110, 120).

16. Instruments according to Claim 15, characterised in that the length of the grooves (53, 54, 55, 56) is determined so as to limit the penetration of the osteotome chisel (90) to a depth which is just sufficient for the anchoring and exact positioning of each of the fins (1110, 1210) corresponding to the rear cortical substance of the vertebra.

17. Instruments according to Claim 14, characterised in that the prosthesis impacter connector (60) consists of a threaded component, in the shape of a parallelepiped, made of soft material, one end of which, opposite the thread (62), is shaped according to the shape of the plates (110, 120) of the prosthesis, and the other end of which is fitted with a strike plate (63) made of hard material.

18. Instruments according to Claim 14, characterised in that the tool for manoeuvring the jigs, the prosthesis impacter connector and the osteotome chisel consists principally of a metal rod (70), one end (71) of which is threaded to the dimensions of the threaded orifice of the slot-cutting jigs, of the impacter connector and of the base (93) of the chisel (90), and the other end of which has a driving square (72) preceded by a semi-circular recess (73) onto which fits a sleeve (74) with a striker end-piece (79), retractable by means of a push rod (75) returned to its position by a spring (76) providing, by means of a moving cylindrical key (77) trapped in an oblong housing (78), the attachment of the said sleeve (74) to the rod (70), by means of the semi-circular recess (73) and tangential grooves (751, 752), of various depths, made in the push rod (75).

19. Instruments according to Claim 14, characterised in that the forceps (80) for fitting the prosthesis (100), the arms (810, 820) of which are normally held apart by a spring (83), is equipped with end-pieces (811, 821) corresponding in shape to the plates (110, 120) of the prosthesis (100), each having two rods (812, 813 and 822, 823), intended to enter the orifices (115, 116, 125, 126) provided for this purpose in the plates (110, 120).

20. Instruments according to Claim 14, characterised in that the prosthesis extractor consists of four rods (910, 920, 930, 940) with a knurled control knob (911, 921, 931, 941) and a threaded end (912, 922, 932, 942), mounted so as to slide in orifices made in a joint control plate (950) serving as a positioning jig with respect to the threaded orifices provided for this purpose in the plates, and as a strike component.

## Patentansprüche

1. Bandscheibenprothese, dadurch gekennzeichnet daß sie hauptsächlich aus zwei Platten besteht (110, 120), die jeweils mit Verankerungsrippen (1110, 1210) versehen und durch ein Gelenkteil (20) getrennt sind, das aus einer sphärischen Kalotte (21) besteht, dadurch gekennzeichnet, daß die sphärische Kalotte (21) eine zylindrische Basis (22) besitzt, die in einen zylindrischen Hohlraum (122) mit gleichem Durchmesser eingesetzt ist, der in der oberen Seite der unteren Platte (120) ausgeführt ist, und daß jede einzelne Platte (110, 120) auf der Rückseite Gewindebohrungen (115, 116, 125, 126) aufweist, die symmetrisch beidseits des zylindrischen Hohlraums (122) und parallel zu den Verankerungsrippen (1110, 1210) angeordnet sind.

2. Bandscheibenprothese nach Anspruch 1, dadurch gekennzeichnet, daß die winkelförmige Aussparung der unteren (120) und oberen (110) Platten durch zwei Flansche mit doppelter Zahnung (113, 123) mit einander ergänzendem Querschnitt begrenzt sind, die sich jeweils auf dem Umfang der oberen Platte (110) und auf dem Umfang der unteren Platte (120) befinden.

3. Bandscheibenprothese nach Anspruch 2, dadurch gekennzeichnet, daß der Flansch mit doppelter Zahnung (123) in der unteren Platte (120) den gesamten Teil einnimmt, der sich zwischen dem Rand des zylindrischen Hohlraums (122) des Gelenkteils (20) und dem Rand der Platte (120) befindet.

4. Bandscheibenprothese nach Anspruch 2, dadurch gekennzeichnet, daß der Flansch mit doppelter Zahnung (113) in der oberen Platte (110) den ganzen Teil einnimmt, der sich zwischen dem Rand des halbrunden Hohlraumes (117) und dem Rand der Platte (110) befindet, wobei er den Aktionsradius des Gelenkteils (20) sichert.

5. Bandscheibenprothese nach Anspruch 4, dadurch gekennzeichnet, daß der Durchmesser (D) des zylindrischen Hohlraumes (122), der den Aktionsradius des Gelenkteils (20) sichert, und die geometrischen Eigenschaften des oberen (113) und des unteren (123) Flansches mit doppelter Zahnung derart festgelegt sind, daß wenn die Flansche (113, 123) sich ineinander verzahnen, die maximale relative Winkelverschiebung alpha der Platten (110, 120) derjenigen entspricht, die zwischen zwei Wirbeln natürlicherweise existiert.

6. Bandscheibenprothese nach Anspruch 3, dadurch gekennzeichnet, daß der Flansch mit doppelter Zahnung (113) der oberen Platte (110) aus der Verbindung einer ringförmigen Fläche (112) gebildet ist, die zu der Basis der Platte (110) in einem Winkel beta von ca. 10° gegenüber dem Rand der Platte geneigt ist, und einer weiteren ringförmigen Fläche (114), ausgehend vom Rand des halbrunden Hohlraumes (117), wobei ein Winkel delta von ca. 70° gegenüber der Basis der Platte (110) gebildet wird.

7. Bandscheibenprothese nach Anspruch 4, dadurch gekennzeichnet, daß der Flansch mit doppelter Zahnung (123) der unteren Platte (120) aus der Verbindung einer ringförmigen Fläche (124) mit einem Winkel theta von ca. 20° gegenüber der Basis der Platte (120) und von einer weiteren ringförmigen Fläche (127) gebildet ist, ausgehend vom Rand des zylindrischen Hohlraumes (122) wobei ein Winkel gamma von ca 60° gegenüber der Basis der Platte (120) gebildet wird.

8. Bandscheibenprothese nach Anspruch 1, dadurch gekennzeichnet, daß die Basis (22) des Gelenkteils (20) auf dem Boden des zylindrischen Hohlraumes (122) mittels eines Rings aus elastischem Material (30) aufliegt, dessen Durchmesser (d) kleiner ist als der (D) der Basis (22) des Gelenkteils (20).

9. Bandscheibenprothese nach Anspruch 1, dadurch gekennzeichnet, daß die Verankerungsrippen (1110, 1210) der Platten (110, 120) an der Basis der Wirbel (400, 410) verjüngt sind und an ihrem Ende ein Relief mit "Sägezähnen" (1111, 1211) bilden.

10. Bandscheibenprothese nach Anspruch 9, dadurch gekennzeichnet, daß die Oberkante der Zähne in derjenigen Richtung nach vorne geneigt ist, in der die Verankerungsrippen in ihre Aussparungen (401, 411) eingeführt werden.

11. Bandscheibenprothese nach Anspruch 1 oder 9, dadurch gekennzeichnet, daß die Stärke (E) der Rippen (1110, 1210) an ihrer Basis etwas größer ist als die Breite (ℓ) der zuvor in den Wirbeln (400, 410) ausgeführten Aussparungen (401, 411).

12. Bandscheibenprothese nach Anspruch 1, dadurch gekennzeichnet, daß die Platten (110, 120) und ihre Rippen (1110, 1210) aus Titan oder Titanlegierung hergestellt sind, wobei alle Flächen, die mit den Wirbeln in Kontakt kommen, auf der Oberfläche mit Plasma behandelt werden.

13. Bandscheibenprothese nach dem Anspruch 1, dadurch gekennzeichnet, daß das Gelenkteil (20) aus Polyethylen hergestellt ist.

14. Vorrichtung für die Implantation der Prothese nach einem der obengenannten Ansprüche, umfassend eine Zange (80) zum Einsetzen der Prothese, dadurch gekennzeichnet, daß sie besteht aus:
- einem Schablonensatz zum Spreizen (50) und Aussparen der Wirbel,
- einem Osteotomie-Meißel (90) zur Ausführung der Aussparungen (401, 411),
- einer Prothesenschlageinrichtung (embout impacteur) (60) mit eingebauter Schlagplatte (63),
- einem Werkzeug zur Betätigung der Schablone (50), der Prothesenschlageinrrichtung (embout impacteur) (60) und des Osteotomie- Meißels (90),
- einem Prothesenextraktor (900).

15. Vorrichtung für die Implantation nach Anspruch 14, dadurch gekennzeichnet, daß die Spreiz- und die Aussparungsschablone (50) aus einer Platte passender Dicke bestehen, mit der gleichen Form und dem gleichen Platzbedarf wie die entsprechenden Platten (100, 120) der Prothese (100), wobei sie an der Vorderseite abgerundete Kanten (51) und von hinten nach vorne eine Gewindebohrung (52) mit den gleichen Abmessungen wie das Gewindeende (71) der Stange (70) des Werkzeuges für die Betätigung der Schablonen (50) und der Schlageinrichtung (60) besitzen, die sich in der Längsmittelebene der Platte befindet, sowie vier Nuten (53, 54, 55, 56), die paarweise symmetrisch beidseitig der Längsmittelebene der Platte in einem Abstand angeordnet sind, der genau demjenigen der Verankerungsrippen (1110, 1210) jeder Platte (110, 120) entspricht.

16. Vorrichtung nach Anspruch 15, dadurch gekennzeichnet, daß die Länge der Nuten (53, 54, 55, 56) so festgelegt ist, daß der Osteotomie-Meißel (90) gerade so tief eindringen kann, daß die Verankerung und die exakte Positionierung jeder Rippe (1110, 1210) möglich ist, entsprechend der hinteren Rindenschicht des Wirbels.

17. Vorrichtung nach Anspruch 14, dadurch gekennzeichnet, daß das Einschlagstück (60) der Prothesen aus einem Gewindestück in Form eines Parallelepipedes aus weichem Material besteht, wobei eines der Enden, das dem Gewindeteil (62) gegenüberliegt, entsprechend der Form der Platten (110, 120) der Prothese geformt ist und das andere Ende mit einer Schlagplatte (63) aus hartem Material versehen ist.

18. Vorrichtung nach Anspruch 14, dadurch gekennzeichnet, daß das Werkzeug zur Betätigung der Schablonen, des Einschlagstückes (embout impacteur) für Prothesen und des Meißels für die Osteotomie hauptsächlich aus einer Metallstange (70) gebildet ist, deren eines Ende (71) entsprechend der Größe der Gewindebohrung der Aussparungseinrichtungen, dem Einschlaggstück (embout impacteur) und der Befestigungseinrichtung (93) des Meißels (90) mit einem Gewinde vershen ist, und deren anderes Ende einen Antriebsvierkant (72) aufweist, der in einem vorderen Teil eine halbkreisförmige Auskehlung (73) besitzt, und auf dem eine Halterung (74) mit Schlagansatzstück (79) angebracht ist, die mit Hilfe eines Stößels (75) einziehbar ist, welcher mittels einer Feder (76) vorgespannt wird, und mittels eines beweglichen zylindrischen Keils (77), der in einer länglichen Vertiefung (78) sitzt, die Verbindung der Halterung (74) mit der Stange (70) mit Hilfe der halbkreisförmigen Auskehlung (73) und tangentialer Rillen (751, 752), die unterschiedlich tief in dem Stößel (75) ausgeführt sind, sichert.

19. Vorrichtung nach Anspruch 14, dadurch gekennzeichnet, daß die Zange zum Einsetzen (80) der Prothese (100), deren Griffpaar (810, 820) normalerweise durch eine Feder (83) gespreizt ist, mit Vorsatzstücken (811, 821) versehen ist, die an die Form der Platten (110, 120) der Prothese (100) angepaßt sind, wobei jede zwei Stangen (812 813 und 822, 823) enthält, die dazu bestimmt sind, in das Innere der Öffnungen (115, 116, 125, 126) einzudringen, die zu diesem Zweck in den Platten (110, 120) vorgesehen sind.

20. Vorrichtung nach Anspruch 14, dadurch gekennzeichnet, daß der Prothesenextraktor aus vier Stangen (910, 920, 930, 940) mit geriffeltem Betätigungsknopf (911, 921, 931, 941) und mit einem Gewindeende (912, 922, 932, 942) besteht, die in Öffnungen, die in eine gemeinsame Betätigungsplatte (950) gebohrt sind, beweglich eingesetzt sind, wobei sie als Positionierungsschablone für die Gewindebohrungen, die zu diesem Zweck in den Platten vorgesehen sind und für die Schlagvorrichtung dienen.
